# EUROPEAN PATENT APPLICATION

(11) **EP 4 406 542 A1**
(43) Date of publication of application: **31.07.2024**
(21) Application number: 23153551.9
(22) Date of filing: 26.01.2023
(51) Int. Cl.: A61K 31/716, A61K 31/722, A61K 31/592, A61K 31/593, A61K 35/66, A61K 35/74, A61K 35/741, A61K 35/744, A61K 35/745, A61K 35/747, A61P 1/00, A61P 31/04

(54) **TREATMENT OF GUT INFLAMMATORY DISEASES**

(71) Applicant: BioKuris, 4040 Herstal (BE)
(72) Inventor: MODICA, Amore Salvatore Michaël, 4420 Saint-Nicolas (BE); DESREUMAUX, Pierre François, 59117 Wervicq-Sud (FR)
(74) Representative: Calysta NV

(57) **Abstract**

Chitosan and/or chitin-glucan for use in the prevention or the treatment of an inflammatory intestinal disease affecting a mammalian patient, the corresponding kit of part and the uses for protecting Vitamin D, or for the prevention and/or treatment of Candida biofilms or gluten-induced inflammations.

## Description

### Technical domain

The present disclosure concerns the use of Chitosan and/or chitin glucan to treat intestinal inflammatory diseases or disorders, or to protect vitamin D in the digestive tract. Accordingly, the present invention further relates to a kit of part comprising Chitosan and/or chitin glucan, and vitamin D and/or a probiotic.

### State of the art

Intestinal inflammatory diseases, such as Crohn's disease and Ulcerative Colitis, affect tens of millions of human patients worldwide, and even animals such as dogs.

These disease cause pain, fever, local inflammations, local lesions, ulcers, weight loss.

In addition, the prevalence of gluten-associated inflammations increases.

Beside chemical molecules or antibodies to directly treat the inflammation (i.e. the consequence of an underlying problem), biological agents such as probiotics, vitamins, or polysaccharides aim either at addressing the cause or the inflammation, in a more gentle and/or physiological way.

Finally, deficiencies in Vitamin D or in intestinal short chain fatty acids become more and more problematic.

Patent BE1024968 discloses a composition comprising chitosan and chitin glucan.

The patent application WO 2007/122187, and the corresponding granted patents disclose chitin glucan for oral administration, in order to provoke several beneficial effects to a patient.

EP2555760 discloses chitosan derivatives, with at least 2% of modified glucosamine residues for the treatment of gastrointestinal diseases.

EP0888118 discloses chitosan oligosaccharide or beta glucan oligosaccharide with a prebiotic for an action in the intestine.

CN110179108 discloses chitosan oligosaccharide or beta glucan oligosaccharide for the homeostasis of the gut flora.

EP2968651 discloses a composition for the treatment of an intestinal lesion comprising a mucoadhesive and a homeostatic agent; both being potentially chitosan.

EP2832352 discloses a capsule comprising chitosan powder. Probiotics or vitamin D are also mentioned in this document.

Unfortunately, although these documents describe the merits of their technology, they do not disclose how to treat inflammatory diseases affecting the intestine.

### Summary of the invention

A first aspect of the present invention is a composition comprising chitosan and/or chitin-glucan for use in prevention or treatment of an inflammatory intestinal disease affecting a mammalian patient, being preferably selected from the group consisting of Irritable Bowel Syndrome (IBS), Inflammatory Bowel Disease (IBD), being preferably Crohn's disease or ulcerative colitis, and gluten intolerance or antibiotic-induced diarrhea.

Preferably, this pharmaceutical composition further comprises Vitamin D.

Preferably, this pharmaceutical composition further comprises a probiotic, preferably a yeast or a bacteria more preferably selected from the group consisting of strains of the genera *Bifidobacterium, Lactobacillus, Lacticaseibacillus,* Leuconostoc or *Ligilactobacillus,* still more preferably *Bifidobacterium* breve, *Lactobacillus* acidophilus, such as the strain NCFM (DSM33840), or the strain La-14 (DSM33880), *Lacticaseibacillus* paracasei, such as the strain Lpc-37 (DSM32661), or *Ligilactobacillus salivarius,* such as the strain Ls-33 (DSM33831).

Advantageously, this composition is administered orally and/or the is in the form a food product, a dietary supplement, or a pharmaceutically acceptable formulation.

A related aspect of the present invention is a kit of part comprising chitin glucan and/or chitosan, and vitamin D and/or a probiotic (preferably as described above).

Preferably, this kit is for oral administration to a mammalian patient, preferably a mammalian patient suffering of an inflammatory intestinal disease, being preferably Irritable Bowel Syndrome (IBS) or Inflammatory Bowel Disease (IBD) or diarrhea.

Another related aspect of the present invention is pharmaceutical composition comprising chitosan and/or chitin-glucan for use in the prevention or the treatment of a Candida sp. (Candida alba) biofilm affecting a patient, this pharmaceutical composition being preferably administered orally, topically, vaginally or rectally.

Advantageously, this pharmaceutical composition is in the form of a kit of parts further comprises a probiotic (bacteria, yeast) as described above and/or a molecule active against Candida *sp*.

Another related aspect of the present invention is pharmaceutical composition comprising chitosan and/or chitin-glucan for use in the prevention and/or the treatment of gluten-induced inflammation, this pharmaceutical composition being preferably administered orally.

Another related aspect of the present invention is pharmaceutical composition comprising chitosan and/or chitin-glucan for use in increasing the production of propionic acid and/or of butyric acid in the intestine of a mammalian patient in need thereof, the said pharmaceutical composition being preferably administered orally.

Another related aspect of the present invention is pharmaceutical composition comprising chitosan and/or chitin-glucan for use in improving Vitamin D absorption by the intestine of a mammalian patient in need thereof (e.g. suffering from intestinal inflammation and/or of diarrhea, and/or lacking of Vitamin D), the said pharmaceutical composition being preferably administered orally.

Another related aspect of the present invention is pharmaceutical composition comprising chitosan and/or chitin glucan for use in the treatment of biliary acids malabsorption in a mammalian patient in need thereof.

### Detailed description of the invention

The inventors have identified beneficial effects of chitin-glucan or of chitosan, especially in synergy with vitamin D and/or probiotics for the treatment of intestinal inflammatory diseases (affecting a mammalian patient).

Therefore, a first aspect of the present invention is a composition, preferably an edible composition, comprising chitosan and/or chitin-glucan for use in the prevention or the treatment of an inflammatory intestinal disease.

Preferably, this composition is edible and is administered orally. When administered orally, chitosan and/or chitin glucan is advantageously formulated for administration of between 10 and 1000 mg/kg (body weight), preferably between 20 and 500 mg/kg, more preferably between 50 and 100 mg/kg.

Other advantageous modes of administration include topical, for instance in a patch or a cream, or as suppositories (and/or rectal administration). Suitable concentrations in these advantageous administration modes range between 2.5 mg/ml and 50 mg/ml, preferably between 5 and 25 mg/ml, more preferably between 10 and 20 mg/ml.

Preferred mammalian patients are Humans or dogs.

In the context of the present invention, preferably, "chitosan" refers to a chitin derivative, wherein a proportion of the N-acetyl-D-glucosamine residues is deaminated. Preferably, the proportion of D-glucosamine (mole D-glucosamine:total of moles D-glucosamine+ moles N-acetyl-D-glucosamine) ranges between 50% and 95%, more preferably between 75% and 90%, such as between 80% and 86%. The patent document BE 2022/5208, filed on March 22, 2022, describes an improved production process of chitosan. Preferred chitosan molecules have less than 20% of glucan residues, advantageously, less than 10% of glucan residues (moles glucan monomers : total monomers of the chitosan, i.e. mole D-glucosamine+ mole N-acetyl-D-glucosamine).

In the context of the present invention, a preferred chitosan molecule has a molecular weight comprised between 5000 and 30000 Da, more preferably between 10000 and 20000 Da, such as about 15000 Da.

In the context of the present invention, preferably, "chitin glucan" refers to a copolymer of chitin and beta (β) glucan. Preferably, this copolymer is extracted from fungal sources. In this copolymer, segments of N-acetyl-D-glucosamine (potentially with minor amounts of D-glucosamine, less than 50%, preferably less than 30%, preferably less than 20%, preferably less than 10%: mole D-glucosamine:total of mole D-glucosamine+ mole N-acetyl-D-glucosamine) are linked with segments consisting of beta (β) glucan (1,3); (1,3)(1,6) and/or (1,3)(1,4) bonds. Preferably, the weight ratios between chitin (mole D-glucosamine+ mole N-acetyl-D-glucosamine) and beta glucan (mole of glucan monomers) range between 5:95 and 95:5, more preferably between 20:80 and 70:30, more preferably between 25:75 and 40:60.

Advantageously, due to its segmented structure, chitin-glucan has a different 3D configuration if compared with chitin or glucan or with a mixture of chitin and glucan (non-segmented) polymers.

Chitosan and chitin-glucan can be found in many different organisms, including animal or fungal species. A convenient source for both chitosan and chitin-glucan is *Aspergillus niger.* The polymers are advantageously extracted as described in WO 2007/122187 and the related patents, or in the patent EP1483299.

In the context of the present invention, preferably, the inflammatory intestinal disease is associated with one or several from (local) pain, local inflammations, local lesions, ulcers and/or necrosis, weight loss, blood presence in fecal samples, arthritis, chronic diarrhea, bloating, buccal ulcers, delayed growths (infants), preferably local pain and either local inflammation or local lesions (including ulcers or necrosis) or both local inflammation and local lesions, and/or is selected from the group consisting of Irritable Bowel Syndrome (IBS), Inflammatory Bowel Disease (IBD), gluten intolerance and antibiotic-induced diarrhea.

In the context of the present invention, preferably, Irritable Bowel Syndrome (IBS) refers to a "disorder of brain-gut interaction" characterized by a group of symptoms that commonly include abdominal pain and/or abdominal bloating and changes in the consistency of bowel movements. IBS can be diarrhea-predominant or, conversely, constipation-predominant, or with mixed/altering stool pattern, or pain-predominant. The primary symptom of IBS is abdominal pain or discomfort in association with frequent diarrhea or constipation and a change in bowel habits.

Alternatively, or in addition, preferably, the patients are considered as suffering from IBS according to the Roma IV criteria (Mearin et al. Gastroenterology 2016), based on (i) chronic abdominal pains (≥ 1 day/week within the 3 preceding months) from more than 6 months, and (ii) at least 2 of the 3 following symptoms: pain in conjunction with defecation, change in stool frequency, change in stool consistency.

In practice, preferably, the patients harboring these criteria with no known IBD (see below) are considered as IBS patients in the present invention. Similarly, IBS-like symptoms associated to an intensive sport practice are also encompassed by the present invention.

In the context of the present invention, the patients harboring such clinical symptoms together with another intestinal pathology (chronic intestinal inflammatory diseases, celiac disease, lactose intolerance, microbial overgrowth syndrome) are preferably considered as being affected by IBS and thus fall under the scope of the present invention and benefit from the invention, even if, more generally, they are sometimes considered as suffering from IBS-like disorders. On the other hand, in the context of the present invention, IBD patients (see below) having IBS-like symptoms are preferably considered as IBD patients ; these cohorts include IBD patients under specific medication

(e.g. anti-inflammatory, immune system suppressors, anti-TNF or anti-integrin α4β7 compounds, ,...) aiming, for instance, at alleviating the IBD symptoms or at delaying the flares, since these treated patients might still be considered as IBS or IBS-like: the compositions of the present invention are particularly well adapted to help these patients, on their own, or in synergy with the (i) anti-inflammatory, (ii) immune suppressors and/or, especially for Crohn's patients (iii) blocking antibodies or small molecules (anti-TNF (alpha), anti-integrin α4β7 or even anti-interleukins; IL-12 and/or -23).

In the context of the present invention, preferably, Inflammatory bowel disease (IBD), refers to a group of (chronic) inflammatory conditions of the colon and small intestine. Among them are Chron's disease and Ulcerative colitis. As above-mentioned, in the context of the present invention, preferably, these patients, even if treated for the IBD and still harboring IBS-like symptoms, will be considered as IBD patients.

Another group of patients suffers from gluten intolerance. As it will be developed here below, the condition is improved in these patients thanks to the pharmaceutical composition of the present invention, especially based on chitosan.

Another group of patients suffers from diarrhea, especially following antibiotic treatments the condition is improved in these patients thanks to the pharmaceutical composition of the present invention, especially further comprising a probiotic (including yeast).

Preferably, the composition of the present invention further comprises Vitamin D.

In the context of the present invention, "Vitamin D" preferably refers to a group of fat-soluble secosteroids. Among them is the cholecalciferol (Vitamin D3) or the ergocalciferol (Vitamin D2). Biologically active metabolites or derivatives of Vitamin D3 (e.g. Calcifediol, Calcitriol) are also encompassed in the present invention.

The Inventors have found that chitin glucan and/or chitosan bind Vitamin D, which protects it during its progress through the digestive tract, allowing a gradual, smooth, release to the patient's cells and tissues, and an improved local benefit for the intestine, especially in the lower parts of the intestine and/or a more potent systemic release.

Preferably, in the present invention, Vitamin D is administered by ingestion, advantageously together with (i) the chitosan and/or chitin glucan and/or (ii) with the probiotic (if present; see below). A preferred administration mode is thus a combination of chitosan or chitin glucan with Vitamin D. This maximizes the "smooth" release effect described here above. In other words, this prolongs the absorption time in the intestine and allows a delivery of Vitamin D through an increased portion of the intestine.

Edible supplementation of Vitamin D, separately from the chitin-glucan and/or the chitosan of the present invention (i.e. not in the same tablet or capsule) is also encompassed, especially in the form of a kit of part, with (i) the chitosan and/or chitin glucan and/or (ii) with the probiotic (if present; see below). Although this formulation somehow increases the complexity for the patient, it allows to better take into account the patient complete profile, including the potential deficiency of Vitamin D, the age of the patient, or the level of Vitamin D supplementation already administered to the patient. In this alternative administration mode, Vitamin D is advantageously administered (orally) substantially together with chitosan orchitin glucan, for instance chitosan or chitin glucan is administered first, then, within less than 2 hours, preferably less than 1 hour, less than 30 minutes, less than 15 minutes, less than 5 minutes, Vitamin D is administered, the full daily dose, or a fraction thereof.

Alternatively, the Vitamin D or its active metabolites can be administered by blood injection, or by suppositories, especially in patients having impaired metabolic (liver, kidney) functions.

In the present invention, the Vitamin D is preferably administered in a therapeutically-effective, and/or in a synergistic amount with chitosan or chitin glucan. However, this vitamin should not be administered at too large quantities. Preferred amounts range between 5 and 100 µg (200-4000 IU, for instance of Vitamin D3) per day (for Human adults), preferably between 10 and 80 µg/day, more preferably between 15 and 50 µg/day.

The composition of the present invention preferably further comprises a probiotic.

In the context of the present invention, preferably, "probiotic" refers to a living microorganism (bacteria or yeast) which is ingested or administered topically (also rectally or vaginally) and exerts a beneficial effect, such as a reduction of inflammatory markers and/or the colonization of mucosae, the skin or of the intestine by pathogenic microorganisms.

Preferred probiotics are selected from the genera *Lactobacillus, Lacticaseibacillus,* Leuconostoc, *Bifidobacterium,* or *Ligilactobacillus.*

Preferred probiotics are thus selected from the species *Bifidobacterium* breve, *Lactobacillus* acidophilus, such as the strain NCFM (DSM33840), or the strain La-14 (DSM33880), *Lacticaseibacillus* paracasei, such as the strain Lpc-37 (DSM32661), *Bifidobacterium* breve, or *Ligilactobacillus salivarius,* such as the strain Ls-33 (DSM33831).

All 4 bacterial strains are commercially available from Dupont Nutrition Biosciences ApS.

All these 4 strains have also been deposited at the Leibniz Institute DSMZ - German Collection of Microorganisms and Cell Cultures; Inhoffenstr. 7 B; D-38124 Braunschweig.

The strain DSM32661 (Lpc-37) has been deposited on October 5, 2017, by DuPont Nutrition Biosciences ApS, Langebrogade 1; P.O. Box 17; 1001 Copenhagen K, Denmark.

The strain DSM33840 (NCFM) has been deposited on March 15, 2021, by DuPont Nutrition Biosciences AsP, Langebrogade 1, 1411 Copenhagen K, Denmark.

The strain DSM33831 (Ls-33) has been deposited on February 23, 2021, by DuPont Nutrition Biosciences ApS, Langebrogade 1, 1411 Copenhagen K, Denmark.

The strain DSM33880 (La-14) has been deposited on June 1, 2021 by DuPont Nutrition Biosciences ApS, Langebrogade 1, 1411 Copenhagen K, Denmark.

Yeast (probiotic) is advantageously administered, in synergy with chitosan and/or chitin glucan and possibly also in synergy with vitamin D, to patients suffering from diarrhea by ingestion, or topically (e.g. for treating Candida *sp*. infections).

Other useful probiotics or strains from the above species are thus encompassed by the present invention and can readily be singled out, for instance in running an *in vitro* test based on the production of inflammatory cytokines and/or of anti-inflammatory cytokines (such as IL-10; mRNA level) and/or the expression of factors modulating (reducing) the pain (e.g. PPAR, such as PPARγ; mRNA level) by a cell line, preferably an intestinal cell line (e.g. HT-29, Caco2), either the potential probiotic alone (e.g. at 10⁷ to 10⁹ CFU/ml), or the probiotic together with chitin glucan (e.g. 5 mg/ml), under basal conditions and/or under inflamed conditions. A preferred probiotic will reduce the production by the intestinal cell line (preferably in the presence of chitin glucan or of chitosan) of one or of several inflammatory cytokines (e.g. IL-1 beta; mRNA level) under inflamed conditions at least two-fold, more preferably at least ten-fold or even at least 50-fold. Conversely, or in addition, a preferred probiotic will increase the production of one or of several anti-inflammatory cytokines (e.g. IL-10; mRNA level) and/or of PPAR (mRNA level, such as PPARγ) by the intestinal cell line (preferably in the presence of chitin glucan or of chitosan) under inflamed conditions at least two-fold, more preferably at least ten-fold or even at least 50-fold.

A related aspect of the present invention is a pharmaceutical composition comprising an edible chitosan and/or chitin-glucan for a gradual release of vitamin D in the intestine of a patient in need thereof.

Advantageously, this pharmaceutical composition for a gradual release of vitamin is administered orally.

Preferably, as mentioned above for the kit of parts, this pharmaceutical composition is administered substantially together with the Vitamin D, for instance firstly the chitosan or chitin glucan is administered, and the Vitamin D is ingested within less than 2 hours, preferably less than 1 hour, less than 30 minutes, less than 15 minutes, less than 5 minutes.

Preferably, the patient in need of the pharmaceutical composition comprising an edible chitosan and/or chitin-glucan for a gradual release of vitamin D in the intestine is suffering of, or suspected to suffer from, an inflammatory intestinal condition and/or of an intestinal disorder or irritation.

Another related aspect of the present invention is a pharmaceutical composition comprising chitosan and/or chitin-glucan for use in the prevention or the treatment of a Candida biofilm affecting a patient.

Preferably, this pharmaceutical composition is administered orally, topically, vaginally or rectally, advantageously at, on, or close to the body parts (skin, rectal mucosa, genital mucosae and/or mucosae of the genitourniatory tract) acting, or potentially acting, as Candida reservoirs. A suitable concentration of chitosan (or chitin glucan) for topical administration is of at least 2.5 mg/ml of the composition, preferably at least 5 mg/ml, more preferably at least 10 mg/ml. Also systemic Candida infections (sometimes associated to septic shock) are advantageously treated by the chitosan or chitin glucan of the present invention, preferably administered by injection.

Advantageously, this pharmaceutical composition comprising chitosan and/or chitin-glucan for use in the prevention or the treatment of a Candida biofilm further comprises additional pharmaceutically-acceptable carriers and/or molecules and/or probiotics (see above, bacteria or yeast) to treat Candida infections, or is administered in synergy with these probiotics and/or molecules to treat Candida infections, such as antibiotics and/or antifungal drugs (ingested, injected or locally administered). Such carriers advantageously (additionally) comprise pharmaceutically-acceptable stabilizers and/or surfactants.

Indeed, the inventors have found that chitosan (and chitin glucan) disturb Candida biofilms, which is a very important property, since Candida embedded in biofilms are very difficult to treat and cause long-lasting detrimental effects, including local irritations. Such biofilms can even harbor and protect other microbiological pathogenic strains, which risk to cause additional problems to the patient.

Another related aspect of the present invention is a pharmaceutical composition comprising chitosan and/or chitin-glucan, preferably chitosan, for the prevention and/or the treatment of gluten-induced inflammation.

Preferably, this pharmaceutical composition is administered orally, advantageously substantially together with a gluten-comprising meal, such as a few minutes (less than one hour, less than 30 minutes, less than 15 minutes, less than 5 minutes) before the patient is starting a lunch with gluten-containing ingredients.

Indeed, the inventors have found that chitosan (or chitin glucan) is able to bind to gliadin, neutralizing its capacity to trigger an allergic reaction. This explains why the chitosan or chitin glucan composition should preferably be administered substantially together with the gluten-comprising meal.

Another related aspect of the present invention is an edible pharmaceutical composition comprising chitosan and/or chitin-glucan for increasing the production of propionic acid and/or of butyric acid by the intestine flora of a mammalian patient in need thereof.

This is especially advantageous for patients in the elderity.

Another related aspect of the present invention is chitosan or chitin glucan for use in the treatment of biliary acid malabsorption, preferably an edible pharmaceutical composition comprising chitosan and/or chitin glucan and/or the composition being preferably administered orally.

Another related aspect of the present invention is a method for the treatment of an intestinal inflammation affecting a mammalian patient, comprising the step to administer to the mammalian patient a pharmaceutically effective amount of chitosan and/or chitin glucan, preferably in the form of an edible composition.

Another related aspect of the present invention is a method for improving the bioavailability of Vitamin D in a mammalian patient, this method comprising the step to administer to the mammalian patient a pharmaceutically effective amount of chitosan and/or chitin glucan, preferably in the form of an edible composition. Advantageously, this method allows a more gradual release, a smoother and/or prolonged and/or sustained absorption of Vitamin D through the intestine.

Another related aspect of the present invention is thus a method for improving a gradual release of vitamin D in the intestine of a patient in need thereof, the said method comprising the step of administering a therapeutically effective amount of a pharmaceutical composition comprising an edible chitosan and/or chitin-glucan, the pharmaceutical composition being preferably administered orally.

Another related aspect of the present invention is a method for reducing gluten intolerance in a mammalian patient, this method comprising the step to administer to the mammalian patient a pharmaceutically effective amount of chitosan (and/or chitin glucan), preferably in the form of an edible composition.

Another related aspect of the present invention is a (therapeutic or cosmetic) method for disturbing Candida biofilms affecting a mammalian patient, this method comprising the step to administer to the mammalian patient a pharmaceutically effective amount of chitosan and/or chitin glucan at, on, or close to the Candida biofilm.

Preferably this method further comprises the step of administering a probiotic (see above, bacteria or yeast), locally or by ingestion.

Another related aspect of the present invention is a method for increasing the production of propionic and/or of butyric acid in the intestine of a mammalian patient, or the blood level of the propionic and/or butyric acids, this method comprising the step to administer to the mammalian patient a pharmaceutically effective amount of chitosan and/or chitin glucan, preferably in the form of an edible composition.

Another related aspect of the present invention is a method for the treatment of biliary acids malabsorption comprising the step of administering (orally) an edible pharmaceutical composition comprising chitosan and/or chitin glucan.

### Examples.-

### Example 1: Aluminum-induced colitis

The tap water has been supplemented with 0.5 mg/kg of aluminum to induce visceral hypersensitivity in rat after 7 days of administration. The quantity of free aluminum in the water was measured on a daily basis by Inductively Coupled Plasma MS. Indeed, high concentrations of chitosan and of Chitin-Glucan can potentially chelate a part of the aluminum, which would affect the relevance of the test; yet the inventors found that the majority of the Al remains free.

Chitin-glucan (CG) or chitosan were then administered at 5 different doses twice a Day by oral gavage: 12.5 mg/kg,25 mg/kg; 50 mg/kg; 75 mg/kg and 100 mg/kg for 28 days.

The addition of CG did not affect the body weight gain during the study.

The visceral pain threshold was measured by colorectal distension after 7, 14, 21 and 28 days. A significant reduction has been measured at D7 for the doses of 75 and 100 mg/kg (twice a Day; i.e. 150 and 200 mg/kg/day). A statistically significant effect has also been observed for these doses, and for the dose of 25 mg/kg twice a Day, at Day 28. The same results compared, on an animal-per animal basis, with the pain threshold at Day zero, show a clearer trend of a pain reduction, even at 12.5 mg/kg (twice a Day), which is more robust at the highest doses of 75 and 100 mg/kg twice a Day. Up to 76% of the Alu-induced pain has been relieved by CG administration.

The same approach was followed with chitosan, instead of CG. There was no effect on weight gain.

The effect on pain were less marked than with CG, with a trend for a reduction at doses of 25 mg/kg (twice a day) and higher, but reaching a statistical significance only in certain cases.

### Example 2 - Proof of Concept

Rats were pretreated for 14 days with CG, chitosan, Desferal or Cholestyramine; in each case twice a day, 50 mg/kg. Then the treatment was prolonged for an additional treatment with or without Al-sensibilization, as in Example 1. All these treatments, except desferal, mitigated the Al-induced pain at Day 7, but, in these experimental settings, the beneficial effect was reduced at Day 14.

### Example 3- synergic compounds; toxicity test

A cellular model with HT-29 and Caco-2 cells has been established.

The inventors have firstly tested on Caco-2 cells the effect of CG or of chitosan (both 10 µg to 1500 µg/ml), alone or combined with Vitamin D at physiological conditions (0.0375 to 0.15 µg/ml). There was no cellular toxicity (test: CCK-8; Sigma-Aldrich).

Then, the inventors applied bacterial lipopolysaccharide (10 ng/ml for HT-29 and 1 mg/ml for Caco-2) to mimic inflammatory conditions and, performed the same tests with CG, chitosan and Vitamin D and, again, noticed no toxicity.

### Example 4 - synergic compounds; inflammatory markers

The condition CG 1500 µg/ml+ Vitamin D3 of the Example 3 has been selected by the inventors. This condition has been supplemented with Bifidobacterium breve (B. breve) at three different amounts: 10⁶ CFUs; 10⁷ CFUs and 10⁸ CFUs. The cells have been stimulated (1, 3, 6 and 24 hours) in basal conditions or after LPS pre-stimulation.

The mRNA level of cytokines or of adhesion molecules has been measured.

In conditions with no LPS pre-stimulations, B. breve alone (10⁸ CFUs) or CG and Vitamin D with 10⁶ CFUs did not reduce the mRNA level of II-1β after 1h. Interestingly, CG, Vitamin D and 10⁷ CFUs or 10⁸ CFUs of B. Breve reduced by about 75% the level of II-1β mRNA after 1h. At 3, 6 and 24 h, all the conditions with CG, Vitamin D and B. breve reduced the II-1β mRNA.

Conversely, IL-10 levels are consistently and very sharply increased by CG, Vitamin D and the three B. breve concentrations, at all the time measured.

TNFα; and II-8 levels are increased by CG, Vitamin D and B. breve and TGFβ levels are reduced.

The inventors have also measured the level of molecules involved in intestinal permeability (ZO-1, Occludin, MUC2 and MUC5A).

The expression levels of ZO-1 are reduced in the CG and Vitamin D conditions. MUC5A mRNA level is sharply increased in all the CG and Vitamin D conditions with the three B. breve concentrations and at all the measured times. MUC2 mRNA level remains at basal conditions, except for the condition CG, Vitamin D and 10⁸ CFUs of B. Breve at 24 hours. MOR mRNA level was sharply increased by CG and Vitamin D at all the B. breve concentrations and at all the time tested, whereas PPARγ remains constant, except a sharp increase only for the condition B. breve 10⁸ CFUs and at 24 hours. These cytokines are considered by the inventors as reflecting the response to pain.

Similarly, after LPS pre-stimulation, the inventors have measured a decrease of the expression level of II-1β mRNA for the B. breve conditions 10⁷ CFUs and 10⁸ CFUs at 6 and 24 hours, and a sharp IL-10 and TNFα expression levels induced by CG and Vitamin D, at all the B. breve concentrations and at all the time tested. TGFβ expression level was decreased in all the B. breve conditions with CG and Vitamin D.

ZO-1 level was reduced by CG and Vitamin D at all the B. breve conditions, whereas MUC5AC levels were strongly and consistently increased by CG and Vitamin D at all the B. breve concentrations and at all the times.

MOR mRNA level was sharply increased by CG and Vitamin D at all the B. breve concentrations and at all the times tested. CG and Vitamin D transiently increased the levels of PPARγ (1h, 3h), and the levels was almost basal at 6h.

As controls, the inventors have tested the effect of CG and Vitamin D on the growth of B. breve and *Lactobacillus acidophilus* (*La*)*,* strain DSM33880. There was no effect, neither a stimulation (e.g. due to the possible use of chitosan or of CG by the microorganism for their metabolism), nor an inhibition.

Then the same experiments have been reproduced using *Lactobacillus acidophilus* (*La*)*,* strain DSM33880. The same trends are observed, but the increase of MUC2 expression was much more pronounced without LPS stimulation. After LPS pre-stimulation, IL-8 levels are now decreased, IL-1β levels are now strongly decreased by CG and Vitamin D, at all the tested La concentrations. Similarly, TGFβ levels are strongly reduced.

PPARα and MOR levels are increased, as for B. breve.

The inventors have then compared the effect of chitosan replacing totally CG (1500 µg/ml chitosan vs 1500 µg/ml CG), or partly (750 µg/ml CG + 750 µg/ml chitosan). CG-effect is more robust than chitosan.

### Example 5 - in vivo study

The inventors have developed a model based on 2,4,6-trinitrobenzene sulfonic acid (TNBS)-induced colitis in rats (Sprague Dauwley); 80 mg/kg. Colorectal distension is measured 14 days prior to the TNBS induction, as well as 21, 35 and 49 days after the TNBS induction.

The bacteria B. breve and La have been formulated in sticks for ingestion; the stability having been validated.

The conditions CG and Vitamin D3 resulted into an increased pain threshold (control: 46.67 ± 1.44 vs 53.00 ± 0.82; pressure in mm Hg), which was further potentiated by *B*. breve (55.00 ± 1.12), but not by *La,* until Day 21. Starting from Day 21, the strains La DSM33880 potentiates the Vitamin D effect, at least as efficiently as B. breve. Other molecules used to treat IBS like Phloroglucinol (Spasfon^{®}, also with Vitamin D) did not increase the pain threshold.

Turning to the macroscopic evaluation (Wallace scoring), the TNBS condition resulted in a score of 2.25, which was reduced upon Vitamin D supplementation (2.00) in synergy with CG, and not improved by B. breve supplementation, even in association with Vitamin D and CG. On the contrary, LA supplementation in synergy with CG and vitamin D improves the Wallace scoring to 1.00, whereas Spasfon^{®}, in synergy with Vitamin D, improves the scoring to 1.56.

Similarly, histological analysis of the inflammation (Ameho) shows a limited improvement of B. breve in synergy with CG and Vitamin D: TNBS+vehicle: 2.00; TNBS+CG & Vitamin D, 1.80 and TNBS+CG+Vitamin D+B. breve: 1.50. Interestingly, the supplementation with LA results into an improvement to 0.57. Again, Spasfon^{®} in synergy with Vitamin D, improves the scoring to 1.56. IL-1 and IL-6 were also strongly decreased for the condition with CG, Vitamin D and LA, whereas B. breve provided only intermediate reductions.

### Example 6 - inhibition of Candida biofilms

The inventors have tested the effect of chitosan or of chitin glucan on Candida biofilms and measured a destruction of these biofilms. The inventors have firstly grown Candida biofilms, using Candida albicans ATCC 10231 strain at an inoculum concentration of 4.25 10³ CFU/ml in 96-well-multiwells together with a Mueller-Hinton broth. After 48 hours, the wells have been rinsed with sterile PBS, then a composition comprising chitosan in the same broth is added on the cells in the wells. After 4 days, the culture medium is removed, rinsed with PBS. Then the multiwell is dried for crystal violet staining and the optical density is measured. Chitosan concentrations of 0.075 mg/ml, 0.15 mg/ml, 0.3 mg/ml, 0.6 mg/ml and 1.25 mg/ml have almost no effect on the biofilms (destruction between 2 and 4%), whereas chitosan concentrations of 2.5; 5 and 10 mg/ml destructed the biofilms at 53, 78 and 91%, respectively.

### Example 7 - production profile of short chain fatty acids

The inventors have used a system for the stimulation of intestinal conditions (ascending colon; transverse colon and descending colon). The equivalent of 1.5 or 4.5 g/day of CG has been applied. The inventors have noticed a modulation of the gut microbial fermentation profiles towards increased production of propionates and butyrates. Butyrate, beside a source of energy, is protective against mucosal inflammation and oxidative status. Propionate is also useful as cholesterol-lowering agent and for its effects on glycemic control.

### Example 8 - effect on the symbiotic gut flora

The inventors have measured the relative abundance of bacteria strains forming the flora of Chron-affected gut, of UC-affected gut and in patients with IBS. Fecal samples of patients treated with GC, Vitamin D and La. There was a tendency of restoration towards non-affected levels.

### Example 9- Gluten allergy

Gliadins (part of the gluten) are responsible of an autoimmune enteropathy (celiac disease) caused by an abnormal immune response in genetically susceptible individuals. The inventors have administered orally chitosan together with gluten-enriched meals to patients, or gluten-enriched meals alone.

The supplementation of chitosan resulted into a decrease of the allergic reaction.

### Example 10 - IBS treatment

Women with IBS (140 IBS with constipation, IBS-C; 60 IBS with diarrhea; IBS-D) have been recruited and placed into four groups: 1.5 g of CG (IBS-C, 30 patients; IBS-D, 70 patients) or 3.0 g of CG (IBS-C, 30 patients; IBS-D, 70 patients). Both CG compositions have been supplemented with 400 IU of Vitamin D3. The mentioned doses have been administered to the patients for 30 days under the control of a gastroenterologist, followed by a 15 days post-treatment.

Then the inventors have summarized the symptomatic and wellness improvements. Pain, gas discomfort, IBS discomfort and bloating discomfort have been reduced in 72, 75, 77 and 79% of the patients, respectively. This is very spectacular. Even sleep quality and fatigue symptoms were reduced in 65 and 60% of the patients, respectively. Wellness sensation was similarly greatly improved with a stress feeling reduced in 73% of the patients, a reduction of an overweight feeling by 71%, of food frustration (69%), satiety feeling (65%), energy level (63%), mood level (59%), fitness level (58%), eating pleasure (58%), morale level (57%), efficacity at work (55%), quality of life (53%) and general health (49%). To sum up, 75% of the patients do consider that the treatment has had a beneficial effect on their health. There was no statistical difference between the 1.5 and the 3.0 regimens, nor between the IBS subtypes.

### Example 11 - other probiotics

The inventors have repeated the tests in varying the probiotics. Among the tested probiotics, *Lactobacillus* acidophilus, strain NCFM (DSM33840), *Lacticaseibacillus* paracasei strain Lpc-37 (DSM32661) and *Ligilactobacillus salivarius*, strain Ls-33 (DSM33831) have provided excellent results.

### Example 12 - BAM treatment

The inventors have found that chitin glucan corrects biliary acids malabsorption upon binding and prevention of their accumulation in the intestine.

## Claims

1. A composition comprising chitosan and/or chitin-glucan for use in prevention or treatment of an inflammatory intestinal disease affecting a mammalian patient.

2. The composition of claim 1, wherein the inflammatory intestinal disease is selected from the group consisting of Irritable Bowel Syndrome (IBS),
Inflammatory Bowel Disease (IBD), being preferably Crohn's disease or ulcerative colitis,
gluten intolerance and antibiotic-induced diarrhea.

3. The composition of claim 1 or 2 further comprising Vitamin D.

4. The composition according to any one of the preceding claims, further comprising a probiotic, preferably a bacteria or a yeast.

5. The composition of claim 4, wherein the probiotic is selected from the group consisting of strains of the genera *Bifidobacterium, Lactobacillus, Lacticaseibacillus,* Leuconostoc or Ligilactobacillus, preferably
*Bifidobacterium* breve,
*Lactobacillus* acidophilus, such as the strain NCFM (DSM33840), or the strain La-14 (DSM33880),
*Lacticaseibacillus* paracasei, such as the strain Lpc-37 (DSM32661), or *Ligilactobacillus salivarius,* such as the strain Ls-33 (DSM33831).

6. The composition according to any one of the preceding claims being administered orally and/or the said composition being in the form a food product, a dietary supplement, or a pharmaceutically acceptable formulation.

7. A kit of part comprising chitin glucan and/or chitosan, and
vitamin D and/or a probiotic.

8. The kit of part of claim 7 wherein the probiotic is as described in claims 4 or 5.

9. The kit of part of claim 7 or 8, being for oral administration to a mammalian patient, preferably a mammalian patient suffering of an inflammatory intestinal disease, being preferably Irritable Bowel Syndrome (IBS) or Inflammatory Bowel Disease (IBD) or diarrhea.

10. A pharmaceutical composition comprising chitosan and/or chitin-glucan for use in the prevention or the treatment of a Candida *sp*. biofilm affecting a patient, the said pharmaceutical composition being preferably administered orally, topically, vaginally or rectally.

11. The pharmaceutical composition of claim 10 being in the form of a kit of parts further comprising a probiotic as defined in claims 4 or 5 and/or a molecule active against Candida sp.

12. A pharmaceutical composition comprising chitosan and/or chitin-glucan for use in the prevention and/or the treatment of gluten-induced inflammation, the said pharmaceutical composition being preferably administered orally.

13. A pharmaceutical composition comprising chitosan and/or chitin-glucan for use in increasing the production of propionic acid and/or of butyric acid in the intestine of a mammalian patient in need thereof, the said pharmaceutical composition being preferably administered orally.

14. A pharmaceutical composition comprising chitosan and/or chitin-glucan for use in improving Vitamin D absorption by the intestine of a mammalian patient in need thereof, the said pharmaceutical composition being preferably administered orally.

15. An edible pharmaceutical composition comprising chitosan and/or chitin glucan for use in the treatment of biliary acids malabsorption in a mammalian patient in need thereof.
